# EUROPEAN PATENT SPECIFICATION

(11) **EP 1 971 341 B1**
(45) Date of publication and mention of the grant of the patent: **28.12.2011**
(21) Application number: 07700329.1
(22) Date of filing: 05.01.2007
(51) Int. Cl.: A61K 31/5375, A61K 45/06, A61K 8/40, A61Q 11/00, A61P 43/00, A61P 1/02

(54) **USE OF MORPHOLINO COMPOUNDS FOR THE TREATMENT OF HALITOSIS**
VERWENDUNG VON MORPHOLINO-VERBINDUNGEN ZUR BEHANDLUNG VON HALITOSE
UTILISATION DE COMPOSÉS MORPHOLINO POUR LE TRAITEMENT DE L'HALITOSE

(30) Priority: 10.01.2006 GB 0600431
(43) Date of publication of application: 24.09.2008
(73) Proprietor: Sinclair Pharmaceuticals Limited, Godalming Surrey GU7 1XW (GB)
(72) Inventor: ATTSTRÖM, Rolf, Valter, CH-3855 Brainz (CH)
(74) Representative: Jappy, John William Graham
(86) International application number: PCT/GB2007/000025
(87) International publication number: WO 2007/080378

(56) References cited:
- EP-A- 1 136 067
- WO-A-02/02128
- WO-A-2006/105196
- WO-A-2006/128270
- US-A- 4 894 221
- US-A- 5 082 653
- US-A1- 2003 103 914
- US-A1- 2005 095 208
- ELWORTHY A J ET AL: "Antimicrobial properties of delmopinol against oral bacteria" LETTERS IN APPLIED MICROBIOLOGY, vol. 20, no. 3, 1995, pages 191-194, XP008066666 ISSN: 0266-8254 cited in the application
- RUNDEGREN, J. ET AL: "Delmopinol interactions with cell walls of Gram-negative and Gram-positive oral bacteria" ORAL MICROBIOLOGY AND IMMUNOLOGY , 10(2), 102-9 CODEN: OMIMEE; ISSN: 0902-0055, 1995, XP008066664
- EDWARDSSON C B ET AL: "MICROBIOLOGY OF EARLY SUPRAGINGIVAL PLAQUE DEVELOPMENT AFTER DELMOPINOL TREATMENT", ORAL MICROBIOLOGY AND IMMUNOLOGY, MUNKSGAARD, COPENHAGEN, DK, vol. 8, no. 1, 1 January 1993 (1993-01-01) , pages 36-41, XP008067768, ISSN: 0902-0055
- QUIRYNEN MARC ET AL: "The impact of periodontal therapy and the adjunctive effect of antiseptics on breath odor-related outcome variables: a double-blind randomized study.", JOURNAL OF PERIODONTOLOGY MAY 2005, vol. 76, no. 5, May 2005 (2005-05), pages 705-712, ISSN: 0022-3492
- FDA submission extract by the Applicant for regulatory approval of a commercial formulation of delmopinol

## Description

### Field of the Invention

The present invention relates to the prevention and treatment of halitosis.

### Background to the Invention

Halitosis is commonly known as "bad breath" and has been defined as an unpleasant or offensive odour emanating from the oral cavity (Roldan et al, J Periodontol. 2005 Jun;76(6):1025-33). Although the cause of halitosis can be non-oral, approximately 90% of all offensive odours originate in the mouth (Delanghe et al, Quintessence Int. 1999 May;30(5):307-10). More specifically, approximately 40% of all malodour is thought to originate from the dorso-posterior region of the tongue (Delanghe et al, *supra*).

The principal components of "bad breath" are Volatile Sulphur Compounds (VSCs), especially hydrogen sulphide, methyl mercaptan and dimethylsuphide, or compounds such as putrescine, cadaverine, butyric and propionic acids. These compounds result from proteolytic degradation of various sulphur-containing substrates in food debris, saliva, blood and epithelial cells, by predominantly anaerobic Gram-negative microorganisms in the oral cavity. Substrates for VSC production include the sulphur-containing amino acids methionine, cysteine and cystine, present in saliva or crevicular fluids (Tonzetich J, J Periodontol. 1977 Jan;48(1):13-20; Persson et al, Oral Microbiol Immunol. 1990 Aug;5(4):195-201). Bacteria such as *P. gingivalis, Actinobacillus actinomycetemcomitans, Fusobacterium nucleatum, Tannerella forsythensis, Prevotella intermedia, Campylobacter rectus, Eubacterium sp* and *Spirochetes* are known to produce VSCs (Tonzetich J, *supra*). Oral infections such as gingivitis and periodontitis have been suggested as possible causes of halitosis (Yaegaki K. and Coil JM., J Can Dent Assoc. 2000 May;66(5):257-61).

Effective management of halitosis includes the use of antimicrobial mouthwashes, oral prophylaxis, professional cleaning and the treatment of oral infections and diseases. Although these methods are widespread in clinical dentistry, there are very few scientific evaluations to verify the effectiveness of the different methods. However, it is accepted that successful treatment of halitosis is associated with the reduction of production of VSCs by bacteria that are capable of utilising sulphur containing substrates, in particular the Gram-negative anaerobes. Quirynen et al (J Periodontol. 2005 May;76(5):705-12) concluded that VSC levels correlate to anaerobic bacteria counts in the saliva.

Roldan *et al* (supra) describes a study to determine the effectiveness of a combined therapeutic approach to managing halitosis. A combination of chlorhexidine and cetylpyridium chloride with zinc lactate was adminstered to subjects presenting halitosis. Total bacterial counts in the subgingiva, saliva and the tongue coating were reduced during the treatment period. In particular, a decrease in the levels and proportions of *Porphyromonas gingivalis* in subgingival plaque, unstimulated saliva and tongue coating was seen. This reduction was closely related to reductions in organoleptic scores, volatile sulphur compound (VSC) levels and the Winkel tongue coating index. The ratio of anaerobic to aerobic bacteria in the tongue coating also decreased during the study. The main adverse effect of the treatment was staining of the teeth.

Fine et al (J Clin Periodontol. 2005 Apr;32(4):335-40) reported that an essential oil mouthwash containing zinc chloride (Tartar Control Listerine® Antiseptic) reduced mean bacterial counts from the dorsum of the tongue and supragingival plaque. The reduction in bacterial load, particularly anaerobic bacteria (particularly Gram-negative anaerobes) and VSC-producing bacteria, was found to be responsible for a reduction in malodour.

US-A-20030103914 defines delmopinol as an antimicrobial agent, which can be used as an oral malodour control agent. Such oral malodour controlling agents can be incorporated in oral care compositions, such as mouthwashes, toothpastes or chewing gums, in which an antibacterial seed or pulp extract from the citrus or vitis plant family is present.

There is a need for further products and techniques for effectively preventing, treating and managing halitosis.

### Summary of the Invention

The present invention is based on the surprising discovery that Delmopinol (3-(4-propyl-heptyl)-4-(2-hydroxyethyl)morpholine), and derivatives thereof, is useful in preventing and treating halitosis. Although Delmopinol (and derivatives thereof) is known to be useful for the removal or inhibition of dental plaque formation, as disclosed in US4894221, it has previously been demonstrated that a delmopinol mouthwash does not significantly affect the numbers or proportions of oral bacteria (Elworthy et al, J Clin Periodontol. 1995 Jul;22(7):527-32). As delmopinol is not known to reduce the oral bacterial load, let alone the load of Gram-negative anaerobes, it is surprising that levels of VSCs decrease following treatment with delmopinol.

According to a first aspect of the invention, a morpholino compound having the general formula (I) 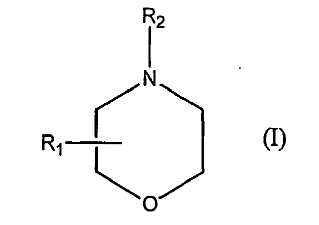 wherein R₁ is a straight or branched alkyl group containing 8 to 16 carbon atoms at the 2- or 3- position of the morpholino ring, and R₂ is a straight or branched alkyl group containing 2 to 10 carbon atoms, substituted with a hydroxy group except in the alpha-position, or a pharmaceutically acceptable salt thereof, is used in the manufacture of a medicament for the prevention or treatment of halitosis.

According to a second aspect of the invention, a kit for use in treating halitosis comprises a compound having the general formula (I), or a medicament containing a compound of formula (I), and instructions that the compound or medicament is to be used for the treatment of halitosis.

### Detailed Description of the Invention

A morpholino compound of formula (I) can be used to treat and prevent halitosis. A morpholino compound according to the invention has the general formula (I) 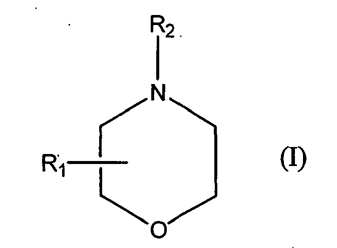 wherein R₁ is a straight or branched alkyl group containing 8 to 16 carbon atoms at the 2- or 3- position of the morpholino ring, and R₂ is a straight or branched alkyl group containing 2 to 10 carbon atoms, substituted with a hydroxy group except in the alpha-position, or pharmaceutically acceptable salts thereof. In a preferred embodiment, the sum of the carbon atoms in the groups R₁ and R₂ of the morpholino compound is at least 10, preferably between 10 and 20. In a further preferred embodiment, the R₂ groupe terminates with the hydroxy group.

The claimed morpholino compounds are known per se and can be manufactured by any known method, for example that disclosed in US5,082,653 and WO90/14342.

The preferred morpholino compound for use in the invention is 3-(4-propyl-heptyl)-4-(2-hydroxyethyl)morpholine, which is commonly known as Delmopinol (CAS No. 79874-76-3).

The morpholino compounds can be used in their free base form or as a pharmaceutically acceptable salt thereof. Examples of pharmaceutically acceptable salts are the salts of acids such as acetic acid, phosphoric acid, boric acid, hydrochloric acid, maleic acid, benzoic acid, citric acid, malic acid, oxalic acid, tartaric acid, succinic acid, glutaric acid, gentisic acid, valeric acid, gallic acid, beta-resorcyclic acid, acetyl salicylic acid, salicylic acid, perchloric acid, barbituric acid, sulfanilic acid, phytic acid, p-nitro benzoic acid, stearic acid, palmitic acid, oleic acid, myristic acid and lauric acid . The most preferred salts are those of hydrochloric acid. A preferred compound is delmopinol hydrochloride (CAS No. 98092-92-3).

As used herein, the term "halitosis" refers to the commonly recognised meaning of the term, i.e. "bad breathe. This may be defined as breath that has an odour that is unpleasant or offensive to the person exhaling the breath, or to others. In a preferred embodiment, the breath contains Volatile Sulphur Compounds (VSC's), including but not limited to hydrogen sulphide, methyl mercaptan or dimethylsuphide, or compounds such as putrescine, cadaverine, butyric and propionic acids. These compounds result from proteolytic degradation of various sulphur-containing substrates in food debris, saliva, blood and epithelial cells, by predominantly anaerobic Gram-negative microorganisms in the oral cavity.

As used herein, the term "treatment" refers to the reduction of halitosis in a patient, i.e. the improvement of the breath odour. Changes in breath odour can be detected by any technique. Preferred techniques include organoleptic detection or, when the breath contains VSCs, the use of a sulfide monitor. Both techniques are well-known in the art, as described in Roldan *et al, supra.*

The current invention is applicable equally in the fields of human and animal medicine, i.e. veterinary applications are within the scope of the invention. In the veterinary embodiment, treatment of pets including cats and dogs, and treatment of farm animals including cattle and swine, are preferred embodiments.

The compound of formula (I) may be brought into contact with the oral cavity in a conventional way, in any suitable form or amount that achieves the desired effect, i.e. reduction of halitosis. Preferably, the compound of formula (I) is in the form of a mouthwash, toothpaste, gel, dentifrice, gum or other similar preparation that will be apparent to the skilled person. The preparation can contain at least one pharmaceutically acceptable excipient. Most preferably, the compound is in the form of an aqueous mouthwash. This can be applied to the oral cavity by the patient, without the need for medical supervision. Preferably, the mouthwash is held in the mouth for at least 5 seconds preferably greater than 10 seconds, for example one minute or more.

In a preferred embodiment, mechanical agitation, preferably brushing or scraping of the tongue, is performed simultaneously with or shortly, preferably immediately, before or after contacting the oral cavity with a compound of formula (I). Most preferably, the compound of formula (I) is applied as an aqueous mouthwash at the start of any regular (e.g. daily) oral health routine, such as before brushing the teeth and tongue.

The compound of formula (I) could be added to an existing toothpaste, gum, gel or mouthwash formulation. The compound of formula (I) may be added in combination with at least one anti-microbial, preferably anti-bacterial, agent. Suitable agents include the antibiotics tetracycline, doxycylcine and ampicillin. Other agents suitable for treating oral infections will be apparent to one skilled in the art.

A treatment of halitosis comprises contacting the oral cavity of a patient that displays symptoms of halitosis with a compound of formula (I), preferably a preparation containing a compound of formula (I) such as a toothpaste, gum, gel or mouthwash formulation. The compound of formula (I) will reduce or remove the halitosis.

Halitosis can be prevented by contacting the oral cavity with a compound of formula (I), preferably a preparation containing a compound of formula (I) such as a toothpaste, gum, gel or mouthwash formulation, prior to the development of offensive odours. The compound of formula (I) will prevent halitosis from occurring in the patient. In the context of a mouthwash, the patient can "gargle" with the mouthwash to ensure all parts of the oral cavity are brought into contact with the active compound.

A kit or pack for use in treating halitosis comprising a compound of formula (I), or a medicament comprising a compound of formula (I), and instructions directing the user to use the compound to treat halitosis, is included in the invention. Preferably, the instructions recite that the compound or medicament is to be used regularly, preferably at least once daily and more preferably every morning and evening, as part of a regular oral health routine.

## Claims

1. Use of a pharmaceutically acceptable salt of a morpholino compound having the general formula (I) 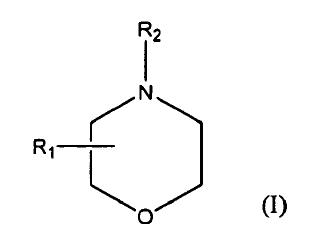 wherein R₁ is a straight or branched alkyl group containing 8 to 16 carbon atoms at the 2- or 3- position of the morpholino ring, and R₂ is a straight or branched alkyl group containing 2 to 10 carbon atoms, substituted with a hydroxy group except in the alpha-position in the manufacture of a medicament for the prevention or treatment of halitosis, wherein the medicament does not contain an antimicrobial agent.

2. Use according to claim 1 , wherein the sum of the carbon atoms in the groups R₁ and R₂ of the morpholino compound is at least 10, preferably between 10 and 20.

3. Use according to claim 1 or 2, wherein R₂ of the morpholino compound terminates with the hydroxy group.

4. Use according to any preceding claim, wherein the morpholino compound is 3-(4-propyl-heptyl)-4-(2-hydroxyethyl) morpholine.

5. Use according to any preceding claim, wherein the medicament further comprises a pharmaceutically acceptable excipient.

6. Use according to any preceding claim, wherein the medicament is in the form of a toothpaste, gum or mouthwash.

7. Use according to any preceding claim, wherein the medicament reduces the level of Volatile Sulphur Compounds emitted from the oral cavity.

8. A kit for use in treating halitosis, comprising a compound as defined in any of claims 1 to 4, or a medicament as defined in any preceding claim, and instructions that the compound or medicament is to be used for the treatment of halitosis.

## Patentansprüche

1. Verwendung eines pharmazeutisch akzeptablen Salzes einer Morpholino-Verbindung mit der allgemeinen Formel (I) 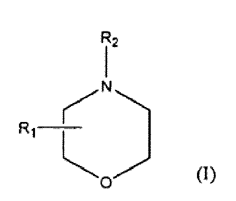 wobei R₁ eine gerade oder verzweigte Alkylgruppe, die 8 bis 16 Kohlenstoffatome enthält, an der 2- oder 3-Position des Morpholino-Rings ist und R₂ eine gerade oder verzweigte Alkylgruppe, die 2 bis 10 Kohlenstoffatome enthält, ist, ersetzt durch eine Hydroxylgruppe außer in der Alpha-Position bei der Herstellung eines Medikaments zur Vorbeugung gegen oder Behandlung von Halitose, wobei das Medikament keinen antimikrobiellen Wirkstoff enthält.

2. Verwendung nach Anspruch 1, wobei die Summe der Kohlenstoffatome in den Gruppen R₁ und R₂ der Morpholino-Verbindung mindestens zehn, vorzugsweise zwischen 10 und 20, beträgt.

3. Verwendung nach Anspruch 1 oder 2, wobei R₂ der Morpholino-Verbindung mit der Hydroxylgruppe endet.

4. Verwendung nach einem vorhergehenden Anspruch, wobei die Morpholino-Verbindung 3-(4-Propylheptyl)-4-(2-hydroxyethyl)morpholin ist.

5. Verwendung nach einem vorhergehenden Anspruch, wobei das Medikament ferner ein pharmazeutisch akzeptables Bindemittel umfasst.

6. Verwendung nach einem vorhergehenden Anspruch, wobei das Medikament in Form einer Zahnpasta, eines Gummis oder einer Mundspülung vorliegt.

7. Verwendung nach einem vorhergehenden Ansprüch, wobei das Medikament die aus der Mundhöhle ausgestoßene Menge flüchtiger Schwefelverbindungen vermindert.

8. Kit zur Verwendung bei der Behandlung von Halitose, der umfasst: eine Verbindung wie in einem der Ansprüche 1 bis 4 definiert oder ein Medikament wie in einem vorhergehenden Anspruch definiert und Anweisungen, dass die Verbindung oder das Medikament zur Behandlung von Halitose zu verwenden ist.

## Revendications

1. Utilisation d'un sel pharmaceutiquement acceptable d'un composé morpholino représenté par la formule générale (I) 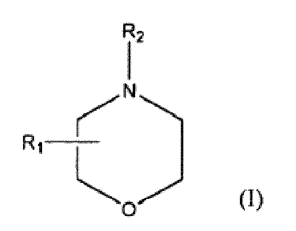 dans laquelle R₁ correspond à un groupe alkyle linéaire ou ramifié contenant 8 à 16 atomes de carbone en position 2 ou 3 du cycle morpholino, et R₂ correspond à un groupe alkyle linéaire ou ramifié contenant 2 à 10 atomes de carbone, substitué par un groupe hydroxy, à l'exception de la position alpha, dans la fabrication d'un médicament pour la prévention ou le traitement de l'halitose, ledit médicament ne contenant pas d'agent antimicrobien.

2. Utilisation selon la revendication 1, dans laquelle la somme des atomes de carbone dans les groupes R₁ et R₂ du composé morpholino est au moins égale à 10 et, de préférence, comprise entre 10 et 20.

3. Utilisation selon la revendication 1 ou 2, dans laquelle R₂ du composé morpholino se termine par le groupe hydroxy.

4. Utilisation selon l'une quelconque des revendications précédentes, dans laquelle le composé morpholino est la 3-(4-propyl-heptyl)-4-(2-hydroxyéthyl) morpholine.

5. Utilisation selon l'une quelconque des revendications précédentes, dans laquelle le médicament contient, en outre, un excipient pharmaceutiquement acceptable.

6. Utilisation selon l'une quelconque des revendications précédentes, dans laquelle le médicament se présente sous la forme d'un dentifrice, d'une gomme ou d'un bain de bouche.

7. Utilisation selon l'une quelconque des revendications précédentes, dans laquelle le médicament réduit le taux de composés sulfureux volatils émis par la cavité buccale.

8. Kit à utiliser pour traiter l'halitose, comprenant un composé tel que défini dans l'une quelconque des revendications 1 à 4, ou un médicament tel que défini dans n'importe laquelle des revendications précédentes, et des instructions indiquant que le composé ou le médicament doit être utilisé pour le traitement de l'halitose.
